# EUROPÄISCHE PATENTANMELDUNG

(11) **EP 2 594 622 A1**
(43) Veröffentlichungstag der Anmeldung: **22.05.2013**
(21) Anmeldenummer: 11009081.8
(22) Anmeldetag: 15.11.2011
(51) Int. Cl.: C10J 3/00, C10L 3/08, C12P 5/02

(54) **Verfahren zur Energieerzeugung mit geschlossenem Kohlendioxid-Kreislauf**

(71) Anmelder: Dr. Pley Environmental GmbH, 96052 Bamberg (DE)
(72) Erfinder: Pley, Martin, Dr., 96117 Memmelsdorf (DE); Cordes, Rudolf, Dipl.-Ing., 49377 Vechta-Langförden (DE)
(74) Vertreter: Ricker, Mathias

(57) **Zusammenfassung**

Verfahren zur Umwandlung von in Phytoplankton gespeicherter Energie in mechanische und/oder elektrische und/oder thermische Energie, wobei das Verfahren die Erzeugung von Kohlendioxid aus Phytoplankton umfasst, dadurch gekennzeichnet, dass erzeugtes Kohlendioxid zur Kultivierung von Phytoplankton verwendet wird.

## Beschreibung

Die vorliegende Erfindung betrifft ein Verfahren zur Umwandlung von in Phytoplankton gespeicherter Energie in mechanische und/oder elektrische und/oder thermische Energie, eine kombinierte Vorrichtung, mit und in welcher das Verfahren betrieben werden kann, sowie die Verwendung des Verfahrens sowie der kombinierten Vorrichtung zur Umwandlung von in Phytoplankton gespeicherter Energie in mechanische und/oder elektrische und/oder thermische Energie.

Zur Energieerzeugung durch Verbrennung werden im großen Umfang fossile Energieträger wie beispielsweise Erdöl und Kohle sowie nachwachsende Rohstoffe wie beispielsweise Holz eingesetzt. Das dabei entstehende Kohlendioxid entweicht zum größten Teil in die Atmosphäre. Hier wirkt es als Treibhausgas und ist somit umweltschädlich.

Eine Aufgabe der vorliegenden Erfindung ist es, ein Verfahren zur Energieerzeugung bereitzustellen, welches umweltfreundlicher als die geschilderten Verfahren ist.

Diese Aufgabe wird gelöst mit einem Verfahren zur Umwandlung von in Phytoplankton gespeicherter Energie in mechanische und/oder elektrische und/oder thermische Energie, wobei das Verfahren die Erzeugung von Kohlendioxid umfasst, welches dadurch gekennzeichnet ist, dass erzeugtes Kohlendioxid zur Kultivierung von Phytoplankton verwendet wird.

Erfindungsgemäß wird dabei bei der Energieumwandlung erzeugtes Kohlendioxid zumindest teilweise, vorzugsweise aber vollständig, zur Kultivierung (Herstellung) von Phytoplankton wiederverwendet, welches wiederum zur Energieerzeugung verwendet werden kann.

Erfindungsgemäß wird als Energieträger Phytoplankton verwendet. Mit Hilfe der Photosynthese baut dieses aus Kohlendioxid und weiteren Nährstoffen seine Körpermasse auf. Diese Körpermasse wird in dieser Offenbarung auch als auch als *"Biomasse"* bezeichnet.

Erfindungsgemäß betrifft die Erfindung somit ein Verfahren zur Umwandlung von in Phytoplankton gespeicherter Energie in mechanische und/oder elektrische und/oder thermische Energie, umfassend mindestens die Stufen (i) bis (vi):
(i) Herstellen von Biomasse umfassend das Beaufschlagen von Phytoplankton mit Kohlendioxid;
(ii) Vergasen der in Stufe (i) hergestellten Biomasse;
(iii) Verbrennen des beim Vergasen der Biomasse in Stufe (ii) erhaltenen Gases und Konvertieren von beim Verbrennen erhaltener thermischen Energie in mechanische und/oder elektrische Energie;
(iv) Oxidieren des beim Verbrennen in Stufe (iii) erhaltenen Abgases;
(v) Abtrennen des Kohlendioxids, welches in den beim Oxidieren in Stufe (iv) erhaltenen Oxidationsprodukten enthalten ist;
(vi) Zuführen des in Stufe (v) abgetrennten Kohlendioxids in Stufe (i) für das Beaufschlagen des Phytoplanktons mit Kohlendioxid.

In einer Ausführungsform wird das in Stufe (i) verwendete Kohlendioxid durch das in Stufe (v) abgetrennte Kohlendioxid zumindest teilweise ersetzt, wobei das im erfindungsgemäßen Verfahren erzeugte Kohlendioxid vorzugsweise in einem geschlossenen Kreislauf geführt wird. Damit wird im erfindungsgemäßen Verfahren kein oder kaum Kohlendioxid in die Atmosphäre freigesetzt, wo es als schädliches Treibhausgas wirken könnte. Dies macht das erfindungsgemäße Verfahren insbesondere auch aus Umweltgründen besonders wertvoll.

Phytoplankton kommt natürlich in stehenden und langsam fließenden Gewässern vor, kann bekanntlich aber auch kultiviert werden, vorzugsweise in Treibhäusern. Es ist deshalb in großen Mengen zugänglich. Aus einer Treibhausfläche von ca. 1 ha können pro Jahr zwischen 100 und 300 to Phytoplankton (berechnet als Trockenmasse) geerntet werden. Die Wachstumsrate ist damit um einen Faktor von ca. 40 höher als die anderer nachwachsender Rohstoffe wie etwa die von Mais oder Holz.

Der Begriff *"Phytoplankton"* wie in dieser Offenbarung verwendet, bedeutet ein Plankton vorzugsweise bestehend aus Algen, Dinoflagellaten, Cyanobakterien, oder einem Gemisch aus zwei oder mehreren davon.

Der Begriff "*Alge*" wie hierin verwendet bedeutet im Wasser lebende eukaryotische, pflanzenartige Lebewesen oder Pflanzen, welche Photosynthese betreiben. Sie können einzellig oder auch mehrzellig sein.

Der Begriff *"Dinoflagellaten"* wie hierin verwendet bedeutet vorwiegend eukaryotische Einzeller.

Der Begriff *"Cyanobakterien"* wie hierin verwendet wird synonym zum Begriff *"Blaualgen*" verwendet.

In einer Ausführungsform sind die Algen ausgewählt aus Kieselalgen, Grünalgen, Goldalgen, Mikroalgen, oder zwei oder mehreren davon.

Der Begriff *"Mikroalge"* wie hierin verwendet bezeichnet eine einzellige Alge oder eine Alge, welche nur wenige Zellen umfasst, vorzugsweise nicht mehr als fünf Zellen.

In einer Ausführungsform umfasst das Phytoplankton die Mikroalge *Chlorella vulgaris.* Diese Alge kann bekanntlich besonders leicht und in großen Mengen kultiviert werden, vorzugsweise in Treibhäusern.

Erfindungsgemäß wird in Stufe (i) Biomasse hergestellt, wobei Phytoplankton mit Kohlenoxid beaufschlagt wird. Durch Photosynthese kommt es zur Vermehrung und Wachstum des Phytoplanktons. Stufe (i) umfasst somit die Kultivierung von Phytoplankton, vorzugsweise in Form von Biomasse.

Nach der Ernte des Phytoplanktons kann dieses getrocknet und/oder pelletiert werden. Falls notwendig kann das geerntete Phytoplankton noch mit einer sauren wässrigen Lösung vorbehandelt werden, um den Anteil an mineralischen Bestandteilen zu minimieren.

Nach der Trocknung wird die Biomasse gemäß Stufe (ii) einem Vergaser zugeführt und dort zumindest teilweise vergast. Vorzugsweise wird das Phytoplankton in Pelletform zugeführt. Vorzugsweise handelt es sich beim Vergaser um einen Festbettvergaser, vorzugsweise um einen Festbettvergaser nach dem Gleichstromprinzip.

In einer Ausführungsform erfolgt das Vergasen in Stufe (ii) dadurch, dass die in Stufe (i) hergestellte Biomasse mit Luft, Sauerstoff, Kohlendioxid oder Wasserdampf, oder einem Gemisch aus zwei oder mehreren davon, umgesetzt wird.

In einer Ausführungsform wird reiner Sauerstoff verwendet.

In einer Ausführungsform wird durch die Zuführung von Luft, Sauerstoff, Kohlendioxid oder Wasserdampf, vorzugsweise durch Zuführen reinen Sauerstoffs, die Biomasse, vorzugsweise in Form von Pellets unterstöchiometrisch und autotherm umgesetzt. Der Begriff *"unterstöchiometrisch"* bedeutet, dass nicht alles oxidierbare Phytoplankton mit Luft, Sauerstoff, Kohlendioxid oder Wasserdampf tatsächlich oxidiert wird. Dabei wird ein Gas, hierin auch als Prozessgas bezeichnet, gewonnen, welches Kohlendioxid, Kohlenmonoxid, Wasserstoff und Kohlenwasserstoffe enthält.

Dieses Prozessgas kann weitere Bestandteile wie Staub und Teerkomponenten enthalten. Zur weiteren Reinigung des Prozessgases können z.B. Teerkomponenten mittels eines FCC (Fluid Catalytic Cracking)-Verfahrens in kurzkettige Komponenten aufgespaltet werden. Dadurch wird der Brennwert des Prozessgases nochmals erhöht. Im Prozessgas enthaltener Staub kann über eine Filterstufe aus dem Gas entfernt werden.

Dem gemäß umfasst in einer Ausführungsform Stufe (ii) mindestens die Stufe (ii'):
(ii') Reinigen des beim Vergasen in Stufe (ii) entstehenden Gases.

In einer Ausführungsform wird das gereinigte Prozessgas über einen Wärmetauscher geleitet. Hierbei wird es auf Raumtemperatur abgekühlt. Dabei werden kondensierbare Bestandteile abgeschieden. Sie können gegebenenfalls für Heizungszwecke verbrannt werden.

Dem gemäß umfasst in einer Ausführungsform Stufe (ii) mindestens die Stufe (ii"):
(ii") Leiten des in Stufe (ii) oder (ii') erhaltenen Gases über einen Wärmetauscher unter Abkühlen des Gases.

Die im Wärmetauscher abgeführte Wärme kann für interne oder externe Prozesse zur Verfügung stehen, vorzugsweise für Heizprozesse. Vorzugsweise kann die Wärme zur Beheizung des Treibhauses verwendet werden, in welchem Phytoplankton kultiviert wird (interner Prozess) oder an externe Verbraucher (externer Prozess).

Das so gereinigte Prozessgas ist ein sogenanntes Schwachgas mit einem Brennwert von 3,5-15 kWh/m³. Es kann in einem Behälter gespeichert werden, vorzugsweise unter Druck.

Gemäß Stufe (iii) wird das beim Vergasen der Biomasse in Stufe (ii) erhaltene Prozessgas verbrannt, wobei die beim Verbrennen erhaltene thermische Energie zumindest teilweise in mechanische und/oder elektrische Energie konvertiert wird.

In einer Ausführungsform wird zur Erzeugung von elektrischer und thermischer Energie mit dem hergestellten Prozessgas eine Verbrennungsmaschine betrieben, bevorzugt ein Gasmotor oder eine Gasturbine oder ein Generator. Derartige Maschinen sind im entsprechenden Stand der Technik bekannt.

Dem gemäß umfasst in einer Ausführungsform Stufe (iii) die Verbrennung des Prozessgases in einem Gasmotor oder einer Gasturbine oder einem Generator.

In einer Ausführungsform durchströmen oder überströmen die Abgase aus der Verbrennungsmaschine, bevorzugt die Abgase aus dem Gasmotor oder der Gasturbine oder dem Generator, einen Katalysator, der geeignet ist, im Abgas enthaltenes Kohlenmonoxid, Kohlenwasserstoffe und Stickoxide zu oxidieren bzw. zu reduzieren. Die Oxidation bzw. Reduktion erfolgt somit vorzugsweise katalytisch. Geeignete Katalysatoren sind aus dem Stand der Technik bekannt, beispielsweise Katalysatoren auf Basis von Edelmetallen und/oder Keramiken. Eine Oxidation oder Reduktion ohne die Verwendung eines Katalysators ist jedoch gleichfalls möglich.

Anschließend kann das nach der Oxidation erhaltene Abgas über einen Wärmetauscher geleitet und auf Raumtemperatur gekühlt werden. Die im Wärmetauscher abgeführte Wärme kann wiederum für interne oder externe Heizprozesse verwendet werden, vorzugsweise für eine Heizung, weiter vorzugsweise zur Beheizung des Treibhauses in welchem Phytoplankton kultiviert wird.

Dem gemäß umfasst in einer Ausführungsform Stufe (iv) mindestens die Stufe (iv'):
(iv') Leiten des beim Oxidieren des beim Verbrennen in Stufe (iii) erhaltenen Abgases erhaltenen Gases über einen Wärmetauscher unter Abkühlen des Gases.

In Stufe (v) wird Kohlendioxid, welches im in Stufe (iv) oder (iv') erhaltenen Gas enthalten ist, aus dem Gas abgetrennt.

In einer Ausführungsform wird über ein angeschlossenes Wäschersystem das im Abgas enthaltene Kohlendioxid aus diesem extrahiert. Vorzugsweise erfolgt die Extraktion mittels N-Methylpyrrolidon nach dem bekannten Purisol-Verfahrens. Das vom Kohlendioxid gereinigte Abgas kann über einen Kamin an die Umwelt abgegeben werden.

In einer Ausführungsform wird der Kohendioxid-haltige Extrakt, vorzugsweise die Kohlendioxid-haltige N-Methylpyrrolidon-Lösung, erhitzt, wobei das Kohlendioxid gasförmig entweicht. Mittels angeschlossener Festbettfilterstufen kann es weiter gereinigt und danach in einem Behälter unter Druck gespeichert werden.

Dem gemäß umfasst in einer Ausführungsform Stufe (v) mindestens die Stufen (v') oder (v') und (v") oder (v') bis (v'"):
(v') Extrahieren von Kohlendioxid aus dem beim Oxidieren in Stufe (iv) erhaltenen Gas;
(v") Erhitzen des in Stufe (v') erhaltenen Extrakts unter Freisetzung von Kohlendioxid;
(v‴) Reinigen des in Stufe (v") freigesetzten Kohlendioxids.

Dieses aus dem Abgas der Stufe (iv) gewonnene Kohlendioxid bzw. das nach den Stufen (v') oder (v') und (v") oder (v') bis (v"') erhaltene Kohlendioxid wird dann wieder als Nährstoff der Phytoplanktonzucht zugeführt, d.h. dieses Kohlendioxid wird bei der Kultivierung von Phytoplankton verwendet.

Vorzugsweise wird im erfindungsgemäßen Verfahren das entstandene Kohlendioxid in einem geschlossenen Kohlendioxid-Kreislauf geführt und umgesetzt. Somit wird bei der erfindungsgemäßen Energieumwandlung kein Kohlendioxid in die Atmosphäre freigesetzt, wo es als umweltschädliches Treibhausgas wirken könnte.

Ein weiterer Gegenstand der Erfindung betrifft eine kombinierte Vorrichtung zur Umwandlung von in Phytoplankton gespeicherter Energie in mechanische und/oder elektrische und/oder thermische Energie, umfassend mindestens folgende Vorrichtungen (i) bis (vi):
(i) eine Vorrichtung zum Herstellen von Biomasse, in welcher Phytoplankton mit Kohlendioxid beaufschlagt werden kann;
(ii) eine Vorrichtung, in welcher die in (i) erhaltene Biomasse vergast werden kann;
(iii) eine Vorrichtung, in welcher das in (ii) erhaltene Gas verbrannt werden und beim Verbrennen erhaltene thermische Energie in mechanische und/oder elektrische Energie konvertiert werden kann;
(iv) eine Vorrichtung, in welcher die in (iii) erhaltenen Abgase oxidiert werden können;
(v) eine Vorrichtung, in welcher das in (iv) erhaltene Kohlendioxid abgetrennt werden kann;
(vi) eine Vorrichtung, in welcher das in (v) erhaltene Kohlendioxid in die Vorrichtung (i) überführt werden kann.

In einer Ausführungsform umfasst die kombinierte Vorrichtung mindestens eine oder mehrere der folgenden weiteren Vorrichtungen:
(i.1) eine Vorrichtung, in welcher das in (i) hergestellte Phytoplankton gereinigt und/oder pelletiert werden kann;
(i.2) eine Vorrichtung, in welcher das in (i) oder (i.1) erhaltene Phytoplankton getrocknet oder getrocknet und pelletiert werden kann;
(ii.1) eine Vorrichtung, in welcher die für das Vergasen in (ii) benötigten Verbindungen bereitgestellt oder hergestellt werden können;
(ii.2) eine Vorrichtung, in welcher das in (ii) oder (ii.1) erhaltene Gas gereinigt werden kann;
(ii.3) eine Vorrichtung, in welche die bei der Reinigung in (ii.2) erhaltenen nichtgasförmigen Produkte überführt werden können;
(ii.4) eine Vorrichtung, in welcher das in (ii) oder (ii.1) oder (ii.2) erhaltene Gas abgekühlt werden kann;
(ii.5) eine Vorrichtung, in welche die in (ii.4) beim Abkühlen des Gases erhaltene thermische Energie überführt werden kann;
(ii.6) eine Vorrichtung, in welcher das in einer oder mehreren der Vorrichtungen (ii) bis (ii.4) erhaltene Gas gespeichert werden kann;
(iv.1) eine Vorrichtung, in welcher das in (iv) erhaltene Gas abgekühlt werden kann;
(iv.2) eine Vorrichtung, in welche die in (iv.1) beim Abkühlen des Gases erhaltene thermische Energie überführt werden kann;
(v.1) eine Vorrichtung, in welche die bei der Abtrennung des Kohlendioxids erhaltenen vorzugsweise flüchtigen Nebenprodukte abgetrennt und/oder überführt werden können;
(v.2) eine Vorrichtung, in welcher das in Stufe (v.1) erhaltene Kohledioxid gereinigt werden kann, d.h. die bei der Abtrennung des Kohlendioxids erhaltenen vorzugsweise festen Nebenprodukte abgetrennt und/oder überführt werden können;
(v.3) eine Vorrichtung, in welcher das in (v) erhaltene Kohlendioxid gespeichert werden kann.

In einer Ausführungsform ist die Vorrichtung
(i) ein Treibhaus;
(ii.4) ein Wärmetauscher;
(ii.5) eine Heizung
(ii.6) ein Druckspeicher;
(iii) ein Gasmotor oder eine Gasturbine oder ein Generator;
(iv) ein Oxidationskatalysator;
(iv.1) ein Wärmetauscher;
(iv.2) eine Heizung;
(v) eine Extraktionsvorrichtung;
(v.1) ein Kamin;
(v.2) ein Festbettfilter;
(v.3) ein Druckspeicher;
(vi) eine Rohrleitung.

Ein weiterer Gegenstand der Erfindung betrifft die Verwendung des erfindungsgemäßen Verfahrens für die Umwandlung von in Phytoplankton gespeicherter Energie in mechanische und/oder elektrische und/oder thermische Energie.

Ein weiterer Gegenstand der Erfindung betrifft die Verwendung der erfindungsgemäßen Vorrichtung für die Durchführung des erfindungsgemäßen Verfahrens.

Ein weiterer Gegenstand der Erfindung betrifft die Verwendung der erfindungsgemäßen Vorrichtung für die Umwandlung von in Phytoplankton gespeicherter Energie in mechanische und/oder elektrische und/oder thermische Energie.

Die **Figur** beschreibt ein Fließschema einer Ausführungsform mit dem geschlossenen Kreislauf des erfindungsgemäßen Verfahrens bzw. der erfindungsgemäßen Vorrichtung. Hierin bedeuten:
(i) eine Vorrichtung zum Herstellen von Biomasse, in welcher Phytoplankton mit Kohlendioxid beaufschlagt werden kann, vorzugsweise ein Treibhaus;
(i.1) eine Vorrichtung, in welche das in (i) hergestellte Phytoplankton überführt und gereinigt und/oder pelletiert werden kann;
(i.2) eine Vorrichtung, in welche das in (i.1) erhaltene Phytoplankton überführt und getrocknet oder getrocknet und pelletiert werden kann;
(ii) eine Vorrichtung, in welche die in (i.2) erhaltene Biomasse überführt und vergast werden kann, vorzugsweise eine Vorrichtung in Form eines Festbetts;
(ii.1) eine Vorrichtung, in welche die für das Vergasen in (ii) benötigten Verbindungen bereitgestellt oder hergestellt werden können, vorzugsweise ein Behälter für Sauerstoff;
(ii.2) eine Vorrichtung, in welche das in (ii) erhaltene Gas überführt und gereinigt werden kann, z.B. eine Crackvorrichtung für Teerprodukte und/oder eine Filteranlage zur Entfernung von Staub;
(ii.3) eine Vorrichtung, in welche die bei der Reinigung in (ii.2) erhaltenen nichtgasförmigen Produkte überführt werden können;
(ii.4) eine Vorrichtung, in welcher das in (ii.2) erhaltene Gas abgekühlt werden kann, vorzugsweise ein Wärmetauscher;
(ii.5) eine Vorrichtung, in welche die in (ii.4) beim Abkühlen des Gases erhaltene thermische Energie überführt werden kann, vorzugsweise eine Heizung;
(ii.6) eine Vorrichtung, in welche das in (ii.4) erhaltene Gas überführt und gespeichert werden kann, vorzugsweise ein Druckspeicher;
(iii) eine Vorrichtung, in welche das in (ii) erhaltene Gas überführt und verbrannt werden kann und beim Verbrennen erhaltene thermische Energie in mechanische und/oder elektrische Energie konvertiert werden kann, vorzugsweise eine Verbrennungsmaschine wie vorzugsweise ein Gasmotor, eine Gasturbine oder ein Generator; der Pfeil symbolisiert die Entnahme von mechanischer und/oder elektrischer und/oder thermischer Energie;
(iv) eine Vorrichtung, in welcher die in (iii) erhaltenen Abgase überführt und oxidiert werden können;
(iv.1) eine Vorrichtung, in welche das in (iv) erhaltene Gas überführt und abgekühlt werden kann, vorzugsweise ein Wärmetauscher;
(iv.2) eine Vorrichtung, in welche die in (iv.1) beim Abkühlen des Gases erhaltene thermische Energie überführt werden kann, vorzugsweise eine Heizung;
(v) eine Vorrichtung, in welche das in (iv.1) erhaltene Gas überführt und das darin enthaltene Kohlendioxid abgetrennt werden kann, vorzugsweise eine Extraktionsvorrichtung;
(v.1) eine Vorrichtung, in welche die bei der Abtrennung des Kohlendioxids erhaltenen vorzugsweise flüchtigen Nebenprodukte abgetrennt und/oder überführt werden können, vorzugsweise ein Kamin;
(v.2) eine Vorrichtung, in welcher die bei der Abtrennung des Kohlendioxids erhaltenen vorzugsweise festen Nebenprodukte abgetrennt und/oder überführt werden können, vorzugsweise ein Staubfilter;
(v.3) eine Vorrichtung, in welche das in (v) erhaltene Kohlendioxid überführt und gespeichert werden kann, vorzugsweise ein Druckspeicher;
(vi) eine Vorrichtung, in welcher das in (v.3) gespeicherte Kohlendioxid in die Vorrichtung (i) überführt wird, vorzugsweise eine Rohrleitung.

Die verschiedenen Vorrichtungen sind vorzugsweise über Rohrleitungen in der im Fließschema angegebenen Fließrichtung verbunden.

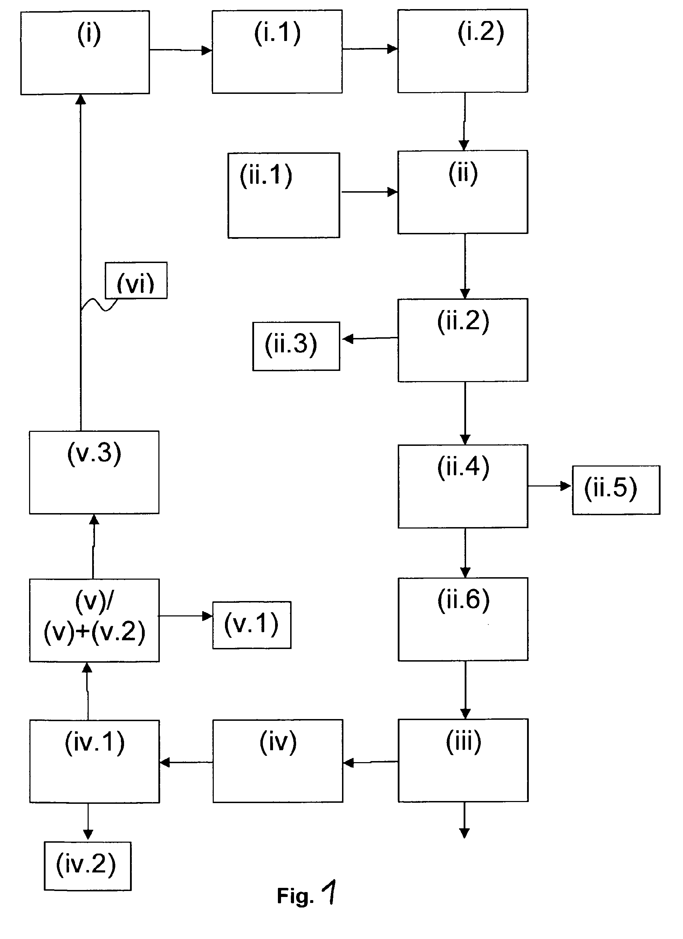

## Patentansprüche

1. Verfahren zur Umwandlung von in Phytoplankton gespeicherter Energie in mechanische und/oder elektrische und/oder thermische Energie, wobei das Verfahren die Erzeugung von Kohlendioxid aus Phytoplankton umfasst, **dadurch gekennzeichnet, dass** erzeugtes Kohlendioxid zur Kultivierung von Phytoplankton verwendet wird.

2. Verfahren nach Anspruch 1, umfassend mindestens die Stufen (i) bis (vi):
(i) Herstellen von Biomasse umfassend das Beaufschlagen von Phytoplankton mit Kohlendioxid;
(ii) Vergasen der in Stufe (i) hergestellten Biomasse;
(iii) Verbrennen des beim Vergasen der Biomasse in Stufe (ii) erhaltenen Gases und Konvertieren von beim Verbrennen erhaltener thermischen Energie in mechanische und/oder elektrische Energie;
(iv) Oxidieren des beim Verbrennen in Stufe (iii) erhaltenen Abgases;
(v) Abtrennen des Kohlendioxids, welches in den beim Oxidieren in Stufe (iv) erhaltenen Oxidationsprodukten enthalten ist;
(vi) Zuführen des in Stufe (v) abgetrennten Kohlendioxids in Stufe (i) für das Beaufschlagen des Phytoplanktons mit Kohlendioxid.

3. Verfahren nach Anspruch 1 oder 2, wobei das Phytoplankton der Stufe (i) ausgewählt ausgewählt ist aus Algen, Dinoflagellaten, Cyanobakterien, oder einem Gemisch aus zwei oder mehreren davon.

4. Verfahren nach Anspruch 3, wobei die Algen ausgewählt sind aus Kieselalgen, Grünalgen, Goldalgen, Mikroalgen, oder zwei oder mehreren davon; oder wobei das Phytoplankton Chlorella vulgaris enthält.

5. Verfahren nach einem der vorstehenden Ansprüche, wobei in Stufe (ii) die in Stufe (i) hergestellte Biomasse mit Luft, Sauerstoff, Kohlendioxid oder Wasserdampf, oder einem Gemisch aus zwei oder mehreren davon, umgesetzt wird.

6. Verfahren nach einem der vorstehenden Ansprüche, wobei Stufe (ii) mindestens die Stufe (ii') umfasst:
(ii') Reinigen des beim Vergasen entstehenden Gases.

7. Verfahren nach einem der vorstehenden Ansprüche, wobei Stufe (ii) mindestens die Stufe (ii") umfasst:
(ii") Leiten des in Stufe (ii) oder (ii') erhaltenen Gases über einen Wärmetauscher unter Abkühlen des Gases.

8. Verfahren nach einem der vorstehenden Ansprüche, wobei in Stufe (iii) die Verbrennung in einem Gasmotor oder einer Gasturbine oder einem Generator stattfindet.

9. Verfahren nach einem der vorstehenden Ansprüche, wobei Stufe (iv) mindestens die Stufe (iv') umfasst:
(iv') Leiten des beim Oxidieren des beim Verbrennen in Stufe (iii) erhaltenen Abgases erhaltenen Gases über einen Wärmetauscher unter Abkühlen des Gases.

10. Verfahren nach einem der vorstehenden Ansprüche, wobei Stufe (v) mindestens die Stufen (v') oder (v') und (v") oder (v') bis (v") umfasst:
(v') Extrahieren von Kohlendioxid aus dem beim Oxidieren in Stufe (iv) erhaltenen Gas;
(v") Erhitzen des in Stufe (v') erhaltenen Extrakts unter Freisetzung von Kohlendioxid;
(v"') Reinigen des in Stufe (v") freigesetzten Kohlendioxids.

11. Kombinierte Vorrichtung zur Umwandlung von in Phytoplankton gespeicherter Energie in mechanische und/oder elektrische und/oder thermische Energie, umfassend mindestens folgende Vorrichtungen (i) bis (vi):
(i) eine Vorrichtung zum Herstellen von Biomasse, in welcher Phytoplankton mit Kohlendioxid beaufschlagt werden kann;
(ii) eine Vorrichtung, in welcher die in (i) erhaltene Biomasse vergast werden kann;
(iii) eine Vorrichtung, in welcher das in (ii) erhaltene Gas verbrannt werden und beim Verbrennen erhaltene thermische Energie in mechanische und/oder elektrische Energie konvertiert werden kann;
(iv) eine Vorrichtung, in welcher die in (iii) erhaltenen Abgase oxidiert werden können;
(v) eine Vorrichtung, in welcher das in (iv) erhaltene Kohlendioxid abgetrennt werden kann;
(vi) eine Vorrichtung, in welcher das in (v) erhaltene Kohlendioxid in die Vorrichtung (i) überführt werden kann.

12. Kombinierte Vorrichtung nach Anspruch 11, weiter umfassend mindestens eine oder mehrere der folgenden Vorrichtungen:
(i.1) eine Vorrichtung, in welcher das in (i) hergestellte Phytoplankton gereinigt und/oder pelletiert werden kann;
(i.2) eine Vorrichtung, in welcher das in (i) oder (i.1) erhaltene Phytoplankton getrocknet oder getrocknet und pelletiert werden kann;
(ii.1) eine Vorrichtung, in welcher die für das Vergasen in (ii) benötigten Verbindungen bereitgestellt oder hergestellt werden können;
(ii.2) eine Vorrichtung, in welcher das in (ii) oder (ii.1) erhaltene Gas gereinigt werden kann;
(ii.3) eine Vorrichtung, in welche die bei der Reinigung in (ii.2) erhaltenen nichtgasförmigen Produkte überführt werden können;
(ii.4) eine Vorrichtung, in welcher das in (ii) oder (ii.2) erhaltene Gas abgekühlt werden kann;
(ii.5) eine Vorrichtung, in welche die in (ii.4) beim Abkühlen des Gases erhaltene thermische Energie überführt werden kann;
(ii.6) eine Vorrichtung, in welcher das in einer oder mehreren der Vorrichtungen (ii), (ii.2), (ii.4) erhaltene Gas gespeichert werden kann;
(iv.1) eine Vorrichtung, in welcher das in (iv) erhaltene Gas abgekühlt werden kann;
(iv.2) eine Vorrichtung, in welche die in (iv.1) beim Abkühlen des Gases erhaltene thermische Energie überführt werden kann;
(v.1) eine Vorrichtung, in welche die bei der Abtrennung des Kohlendioxids erhaltenen vorzugsweise flüchtigen Nebenprodukte überführt werden können;
(v.2) eine Vorrichtung, in welcher das in (v) abgetrennte Kohlendioxid gereinigt werden kann;
(v.3) eine Vorrichtung, in welcher das in (v) oder (v.2) erhaltene Kohlendioxid gespeichert werden kann.

13. Kombinierte Vorrichtung nach Anspruch 12, wobei
(i) ein Treibhaus ist;
(ii.4) ein Wärmetauscher ist;
(ii.6) ein Druckspeicher ist;
(iii) ein Gasmotor oder eine Gasturbine oder ein Generator ist;
(iv) ein Oxidationskatalysator ist;
(iv.1) ein Wärmetauscher ist;
(v) eine Extraktionsvorrichtung ist;
(v.1) ein Kamin ist;
(v.2) ein Festbettfilter ist;
(v.3) ein Druckspeicher ist;
(vi) eine Rohrleitung ist

14. Verwendung eines Verfahrens nach einem der Ansprüche 1 bis 10 für die Umwandlung von in Phytoplankton gespeicherter Energie in mechanische und/oder elektrische und/oder thermische Energie.

15. Verwendung einer kombinierten Vorrichtung nach einem der Ansprüche 11 bis 13 zur Durchführung eines Verfahrens wie in einem der Ansprüche 1 bis 10 definiert; oder Verwendung einer kombinierten Vorrichtung nach einem der Ansprüche 11 bis 13 für die Umwandlung von in Phytoplankton gespeicherter Energie in mechanische und/oder elektrische und/oder thermische Energie.
